# EUROPEAN PATENT APPLICATION

(11) **EP 1 600 157 A1**
(43) Date of publication of application: **30.11.2005**
(21) Application number: 04705180.0
(22) Date of filing: 26.01.2004
(51) Int. Cl.: A61K 31/18, A61K 9/14, A61K 9/20, A61P 13/00, A61P 43/00

(54) **ENTERIC SUSTAINED-RELEASE FINE PARTICLES FOR TAMSULOSIN OR ITS SALT AND PROCESS FOR PRODUCING THE SAME**

(30) Priority: 27.01.2003 US 442984 P
(71) Applicant: Astellas Pharma Inc., Chuo-ku, Tokyo (JP)
(72) Inventor: TANIJIRI, Yoji, Yamanouchi Pharmaceutical Co. Ltd., Yaizu-shi, Shizuoka 425-0072 (JP); ITO, Akira, Yamanouchi Pharmaceutical Co., Ltd., Yaizu-shi, Shizuoka 425-0072 (JP); SUGAO, Hiroya, Yamanouchi Pharmaceutical Co., Ltd., Yaizu-shi, Shizuoka 425-0072 (JP); TAMURA, Tetsuya, Yamanouchi Pharmaceutical Co. Ltd, Yaizu-shi, Shizuoka 425-0072 (JP); NISHIURA, Mare, Yamanouchi Pharmaceutical Co. Ltd., Yaizu-shi, Shizuoka 425-0072 (JP); YAMAZAKI, Shigeru, Yaizu-shi, Shizuoka 425-0072 (JP); MIZUMOTO, Takao, Yaizu-shi, Shizuoka 425-0072 (JP)
(74) Representative: HOFFMANN EITLE
(86) International application number: PCT/JP2004/000644
(87) International publication number: WO 2004/066991

(57) **Abstract**

The present invention relates to enteric sustained-release fine particles of tamsulosin or its salt that can be contained in tablets that disintegrate in the buccal cavity and a manufacturing method thereof. In further detail, the present invention relates to enteric sustained-release fine particles for tablets that disintegrate in the buccal cavity, which comprise (1) tamsulosin or its salt and at least (2) an enterosoluble substance, and when necessary contain (3) a water-insoluble substance, and which have the following characteristics:
1) a particle diameter of approximately 5 to 250 µm
2) when dissolution tests are performed on tablets that disintegrate in the buccal cavity containing these particles by dissolution testing methods cited in the Japanese Pharmacopoeia,
   a) the dissolution rate of tamsulosin or its salt at a pH of 1.2 two hours after starting tests is 25% or less
   b) the time when 50% of the tamsulosin or its salt has dissolved at a pH of 6.8 is 0.5 to 5 hours,
and a manufacturing method thereof.

## Description

### Field of the Invention

The present invention relates to enteric sustained-release fine particles of tamsulosin or its salt that can be contained in tablets that disintegrate in the buccal cavity, and a manufacturing method thereof In further detail, the present invention relates to enteric sustained-release fine particles for tablets that disintegrate in the buccal cavity, which comprise (1) tamsulosin or its salt and at least (2) an enterosoluble substance, and when necessary contain (3) a water-insoluble substance, and which have the following characteristics:
1) A particle diameter of approximately 5 to 250 µm
2) When dissolution tests are performed on tablets that disintegrate in the buccal cavity containing these particles by dissolution testing methods cited in the Japanese Pharmacopoeia,
   a) the dissolution rate of tamsulosin or its salt at a pH of 1.2 two hours after starting tests is 25% or less
   b) the time when 50% of the tamsulosin or its salt has dissolved at a pH of 6.8 is 0.5 to 5 hours
and a manufacturing method thereof.

### Background of the Invention

Tamsulosin and its salts have excellent α₁ receptor-blocking activity and are useful drugs as remedies for the dysuria and the like that accompany prostatomegaly. It is reported that when their release is not controlled, these drugs produce adverse effects in the form of orthostatic hypotension attributed to a sudden rise in the plasma concentration. The pharmaceutical preparations containing tamsulosin hydrochloride that are being marketed today were developed as enteric capsule pharmaceutical preparations comprising enteric sustained-release particles of tamsulosin hydrochloride with which, based on this knowledge, drug release in the early stages following administration is adequately controlled and sudden drug release is avoided, with the drug gradually released over the 5 to 6 hours that follow administration. Furthermore, the enteric granules contained in this capsule preparation have an average particle diameter of 350 to 1,000 µm, and the results of dissolution tests of the capsule preparation performed by the inventors in accordance with the methods cited in the Japanese Pharmacopoeia are as shown below. The two-hour value is approximately 5% when turned 50 rounds by the paddle method using 1 st fluid (pH 1.2) of the Japanese Pharmacopoeia Dissolution Testing Method and the 1.5-hour value is approximately 44% when turned 100, rounds by the paddle method using 2nd fluid (pH of 6.8) of the Japanese Pharmacopoeia:

On the other hand, the importance of improved patient compliance is being advocated. Various tablets that disintegrate in the buccal cavity quickly are being developed so that drugs can be easily taken without water by patients with weak swallowing force, including the elderly, children, and the like, and providing sustained releasability is also in demand as a result of an increase in the number of appropriate drugs.

The following are reports of technologies that provide tablets that disintegrate in the buccal cavity with sustained releasability: Tablets that disintegrate in the buccal cavity are described in US Patent Application No. 6,413,549. These tablets contain drug particles and are manufactured by freeze-drying methods. This text discloses that the drug release speed becomes faster as the particles become smaller, and that uniform coating is possible when the average particle diameter is approximately 500 µm, for instance, when particle size is 75 to 400 µm, but uniform coating of particles of 100 µm or smaller becomes difficult.

A method of tumbling granulation that can be used for tablets that disintegrate in the buccal cavity and a method of manufacturing spherical fine particles with an average particle diameter of 60 to 200 µm by appropriate coating in order to provide sustained releasability and the like are described in International Publication Pamphlet WO00/24379, and coating with conventional coating bases that are presented in various references is disclosed. Moreover, the main objective that particles that have been appropriately treated for sustained release can be contained in tablets that disintegrate in the buccal cavity is found in both Japanese Kokai Patent No. 11-35451 and International Publication Pamphlet WO95/20380.

However, there is no mention of enteric sustained-release fine particles that can be used for tablets that disintegrate in the buccal cavity disclosing specific means intended for use with tamsulosin or its salts, and using tamsulosin for these fine particles is not discussed or disclosed in any reference.

Consequently, there is no knowledge of enteric sustained-release fine particles having dissolution profile appropriate for tamsulosin or its salt that are fine particles with the average particle diameter of approximately 5 to approximately 250 µm that can be used for tablets that disintegrate in the buccal cavity.

### Disclosure of the Invention

Under these conditions, the inventors focused on studies of enteric sustained-release fine particles comprising tamsulosin or its salt that can be used for tablets that disintegrate in the buccal cavity. It was possible to reduce the gritty feeling in the buccal cavity when tablets that disintegrate in the buccal cavity contain fine particles with an average particle diameter of approximately 5 µm to approximately 250 µm. However, it was very difficult to control dissolution of the drug because the fine particles had a large surface area. That is, when dissolution during the early stages of dissolution tests was inhibited, eventual dissolution of the drug during the final stages of the dissolution tests was also inhibited, but when the intention was to increase dissolution during the final stages of the dissolution tests, sufficient inhibition of dissolution during the early stages of dissolution tests was not accomplished. Thus, the object of using tamsulosin or its salt in enteric sustained-release fine particles with an average particle diameter of approximately 5 µm to approximately 250 µm that can be used for tablets that disintegrate in the buccal cavity became clear for the first time, and the goal became to control dissolution of fine particles that are smaller than ordinary granule preparations, which have an average particle diameter of 350 to 1,000 µm, and in particular, to obtain a dissolution profile that is appropriate for tamsulosin and its salts. In light of the fact that, taking into consideration the dissolution profile of commercial capsule preparations, it is necessary to prevent sudden dissolution in order to control the adverse effect of orthostatic hypotension, an object of the present invention is to realize a 50%-dissolution time of two hours, or to prevent further dissolution, with test fluid at a pH of 6.8. Specifically, an object is to realize a dissolution rate at a pH of 1.2 two hours after starting the test of 25% or less and to realize a 50%-dissolution time at a pH of 6.8 of 0.5 to 5 hours.

As a result of performing intense studies that would accomplish these purposes, the inventors successfully inhibited dissolution during the early stages of dissolution tests but avoided excess inhibition of dissolution during the final stages of dissolution tests of fine particles comprising tamsulosin or its salt with an average particle diameter of approximately 5 µm to approximately 250 µm by selecting the appropriate mechanism for controlling drug dissolution.

The present invention was completed as a result of confirming that when these enteric sustained-release fine particles are contained in tablets that disintegrate in the buccal cavity, good disintegration in the buccal cavity, tablet hardness, and dissolution and the like are realized.

That is, the present invention relates to
1. enteric sustained-release fine particles for tablets that disintegrate in the buccal cavity, which comprise (1) tamsulosin or its salt and at least (2) an enterosoluble substance, and when necessary contain (3) a water-insoluble substance, and which have the following characteristics:
   1) A particle diameter of approximately 5 to 250 µm
   2) When dissolution tests are performed on tablets that disintegrate in the buccal cavity containing these particles by dissolution testing methods cited in the Japanese Pharmacopoeia,
      a) the dissolution rate of tamsulosin or its salt at a pH of 1.2 two hours after starting tests is 25% or less
      b) the time when 50% of the tamsulosin or its salt has dissolved at a pH of 6.8 is 0.5 to 5 hours;
2. the enteric sustained-release fine particles for tablets that disintegrate in the buccal cavity according to above-mentioned 1, wherein the enterosoluble substance is an enterosoluble polymer and/or higher fatty acid;
3. the enteric sustained-release fine particles for tablets that disintegrate in the buccal cavity according to above-mentioned 2, wherein the water-insoluble substance is a water-insoluble polymer and/or wax;
4. the enteric sustained-release fine particles for tablets that disintegrate in the buccal cavity according to above-mentioned 3, characterized in that dissolution of the tamsulosin or its salt is controlled by a controlling film and/or matrix;
5. the enteric sustained-release fine particles according to above-mentioned 4, wherein a layer or matrix containing an enterosoluble base is the layer that touches the dissolution fluid or the outermost layer, and the layer containing the water-insoluble substance is farther inside the particles than at least the layer of the enterosoluble base; and
6. a method of producing enteric sustained-release fine particles for tablets that disintegrate in the buccal cavity, which comprise (1) tamsulosin or its salt and at least (2) an enterosoluble substance, and when necessary contain (3) a water-insoluble substance, and which have the following characteristics:
   1) A particle diameter of approximately 5 to 250 µm
   2) When dissolution tests are performed on tablets that disintegrate in the buccal cavity containing these particles by dissolution testing methods cited in the Japanese Pharmacopoeia,
      a) the dissolution rate of tamsulosin or its salt at a pH of 1.2 two hours after starting tests is 25% or less
      b) the time when 50% of the tamsulosin or its salt has dissolved at a pH of 6.8 is 0.5 to 5 hours.

The "enterosoluble substance" of the present invention means an enterosoluble polymer or other enterosoluble base that will at least partially dissolve at a pH of, for instance, 5.0 to 9.0, preferably a pH of 5.5 to 7.5. The enterosoluble polymer is specifically an enterosoluble cellulose derivative, such as hydroxypropylmethyl cellulose acetate succinate, hydroxypropylmethyl cellulose phthalate, hydroxymethylethyl cellulose phthalate, and carboxymethylethyl cellulose, or an enterosoluble acrylic copolymer, such as methacrylic acid-methyl methacrylate copolymer (for instance, brand names of Eudragit L100 and Eudragit S, both made by Röhm GmbH), and methacrylic acid-ethyl acrylate copolymer (for instance, brand names of Eudragit L100-55 and Eudragit L30-D55, both made by Röhm GmbH), and higher fatty acids, such as lauric acid, myristic acid, palmitic acid, and stearic acid, are given as other enterosoluble bases. Of these, methacrylic acid-ethyl acrylate copolymer and stearic acid are preferred. It is possible to use a combination of two or more of these enterosoluble substances. Furthermore, it is possible not only to realize sustained releasability, but also to more easily realize the quality of being enterosoluble, by adding as needed a pH-independent water-insoluble substance such as those listed below:

There are no special restrictions to the "water-insoluble substance" of the present invention as long as it is a water-insoluble polymer or wax that is appropriate for coating fine particles. Specific water-insoluble polymers are water-insoluble cellulose ether, such as ethyl cellulose and Aquacoat (brand name, Asahi Kasei Corp.), water-insoluble acrylic acid copolymers, such as ethyl acrylate-methyl methacrylate-chlorotrimethylammonium ethyl methacrylate copolymer (for instance, brand name: Eudragit RS, Röhm GmbH) and methyl methacrylate-ethyl acrylate copolymer (for instance, brand name: Eudragit NE30D, Röhm GmbH), and the like. Water-insoluble cellulose ether, such as ethyl cellulose and Aquacoat, is particularly preferred.

Solid fats and oils, such as hydrogenated castor oil, hydrogenated coconut oil, and beef tallow, and higher alcohols, such as cetyl alcohol and stearyl alcohol, are examples of the waxes of the present invention.

One or a combination of two or more of these polymers and waxes can also be used for the purpose of controlling dissolution.

In addition, water-soluble polymers, saccharides, salts, and the like can be mixed with the above-mentioned water-insoluble polymers or waxes and the like in order to facilitate control of drug dissolution from the fine particles that have been coated by these substances. Hydroxypropyl cellulose, hydroxypropylmethyl cellulose, polyvinyl pyrrolidone, polyvinyl alcohol, and the like are given as these water-soluble polymers, maltose, maltitol, and the like are given as these saccharides, and sodium chloride and the like are given as these salts. The amount of polymers and saccharides and the like that is used here can be adjusted as needed in order to control the dissolution speed of the drug. Moreover, one or a combination of two or more of these polymers and saccharides and the like can also be used.

Plasticizer can be added in order to improve film-forming capability. Triacetin, triethyl citrate, dibutyl sebacate, acetylated monoglyceride, and Eudragit NE30D (brand name; Röhm GmbH) are examples of this plasticizer, and triacetin and Eudragit NE30D are preferred.

The "enteric sustained-release fine particles" of the present invention means sustained-release fine particles that are enterosoluble comprising tamsulosin or its salt and an enterosoluble substance, and they can further comprise water-insoluble substance as necessary. Moreover, dissolution in the early stages of dissolution tests was inhibited and excess inhibition of dissolution during the final stages of dissolution tests was avoided with fine particles having an average particle diameter of approximately 5 µm to approximately 250 µm comprising tamsulosin or its salt by using the mechanism for controlling drug dissolution from enteric sustained-release fine particles of the present invention that is described below:

The following three embodiments will be considered as the mechanism for controlling drug dissolution from enteric sustained-release fine particles of the present invention:
a. Film-controlled type
b. Matrix type
c. Combination type of film-controlled type and matrix type

The "film-controlled type" means the type of pharmaceutical preparation with which drug dissolution is "controlled by a controlling film" and is one where the drug is covered by a dissolution-controlling film of a polymer and the like. An example is a mechanism for controlling drug dissolution with which polymer and the like is coated over a layer comprising the drug and dissolution of the drug is controlled by the film of the polymer and the like.

The "matrix type" means the type of pharmaceutical preparation with which drug dissolution is "controlled by a matrix" and is one where the drug is dispersed in a base, such as a polymer or wax. An example is a mechanism for controlling drug dissolution with which drug dissolution is controlled by holding the drug in the network structure of the polymer and the like.

The "combination type" means the type of pharmaceutical preparation with which dissolution of the drug is "controlled by a controlling film and matrix" and is one that uses both the film-controlled type and the matrix type. An example is a mechanism for controlling drug dissolution with which dissolution of the drug is controlled by both matrix type and film-controlled type thereafter.

Enteric sustained-release fine particles of the film-controlled type can be obtained by preparing fine particles with sustained releasability of the film-controlled type and then making these fine particles enterosoluble. For instance, sustained-release fine particles are obtained by coating a drug on commercial microcrystalline cellulose grains (Avicel, Asahi Kasei Corp., brand name: Celphere 102, and the like) as the core using a conventional coating device for fluidized bed coating, tumbling fluidized coating, and the like, and further coating this with a water-insoluble polymer or wax to form an film that controls dissolution. Moreover, it is also possible to obtain sustained-release fine particles by preparing a core comprising a drug by high shear granulation, tumbling fluidized granulation, or spray drying and then coating this with a water-insoluble polymer or wax to form a film that controls dissolution. Furthermore, enteric sustained-release fine particles of the film-controlled type can be obtained by coating an enterosoluble substance on the sustained-release fine particles that have been obtained using the above-mentioned coating device. Obtaining enteric sustained-release fine particles of the film-controlled type is not limited to these methods, but the preferred film-controlled type does have a layer comprising water-insoluble substance on the inside and a layer comprising enterosoluble substance on the outside.

For instance, as disclosed in Japanese Kokoku Patent No.7-72129, enteric sustained release particles of the matrix type can be obtained by, for instance, adding enterosoluble substance to a drug and microcrystalline cellulose and performing high shear granulation or tumbling fluidized granulation. Moreover, these particles can be obtained by spray drying, spray cooling, and the like, a solution, suspension, or melt of a drug and enterosoluble substance using an appropriate device, such as a spray dryer. These particles can also be obtained by coating a solution, suspension, or melt of a drug and an enterosoluble substance around a core using a conventional coating device for fluidized bed coating, tumbling coating, and the like. A water-insoluble substance can also be added as needed.

Enteric sustained-release fine particles of the combination type can be obtained by preparing sustained-release fine particles comprising a drug and water-insoluble substance and the like by, for instance, high shear granulation, tumbling fluidized granulation, spray drying, or spray cooling and then coating this with an enterosoluble substance to form a film that controls dissolution. Moreover, it is also possible to coat a core with a solution or suspension of a drug and water-insoluble substance to form sustained-release fine particles and then coat these with an enterosoluble substance.

Taking into consideration the size of the sustained-release fine particles (average particle diameter of approximately 250 µm or smaller), in addition to commercial microcrystalline cellulose grains, for instance, a conventional crystalline filler of approximately 5 µm to approximately 150 µm, specifically crystalline lactose, granular sugar, sodium chloride, corn starch, and the like, can be used as the core used by the above-mentioned method. In this case, a core that has been pre-coated by water-soluble polymer, water-insoluble polymer, and the like in order to round the edges of the filler that serves as the core can also be used. The solvent that is used to prepare these sustained-release fine particles is, for instance, water or an organic solvent. Examples of the organic solvent are alcohols, specifically methanol, ethanol, propanol, isopropanol, and the like, halogenated alkanes, specifically dichloromethane, chloroform, chloroethane, trichloroethane, carbon tetrachloride, and the like, ketones, specifically acetone, methyl ethyl ketone, and the like, nitriles, specifically acetonitrile and the like, and hydrocarbons, specifically n-hexane, cyclohexane, and the like. One of these organic solvents or a mixture of two or more at an appropriate ratio can be used, and a mixture at an appropriate ratio with water can also be used.

The "tablets that disintegrate in the buccal cavity" of the present invention means a pharmaceutical preparation that is tablets or similar to tablets with which disintegration in the buccal cavity occurs within a specific amount of time, specifically in less than 2 minutes, preferably in less than 1 minute. Specific examples are those disclosed in International Publication Pamphlet WO98/02185, International Publication Pamphlet WO95/20380, Japanese Kokai Patent No. 10-182436, Japanese Kokoku Patent No. 5-500956, Japanese Kokoku Patent No. 62-50445, and Japanese Patent No. 287346.

The enteric sustained-release fine particles of tamsulosin or its salt and manufacturing method thereof of the present invention will now be described in detail:

The drug used in the present invention is tamsulosin or its salt and both the free form and salts that are pharmaceutically acceptable can be used. Specific pharmaceutically acceptable salts of tamsulosin are salts of inorganic acids, such as hydrochloric acid, hydrobromic acid, sulfuric acid, nitric acid, and phosphoric acid, and salts of organic acids, such as formic acid, acetic acid, succinic acid, oxalic acid, fumaric acid, maleic acid, lactic acid, malic acid, citric acid, tartaric acid, glutamic acid, and aspartic acid. Hydrochlorides are particularly preferred.

The amount of drug that is added is the amount that is effective in terms of treatment, preferably 0.1 mg to 0.8 mg.

Moreover, other drugs can also be added within a range that does not interfere with the results of the present invention.

The drug is appropriately treated for sustained release and contained in the enteric sustained-release fine particles such that they are fine particles with which release of the drug is controlled. There are no special restrictions to the particle diameter of these enteric sustained-release fine particles as long as it is within a range with which the gritty sensation in the buccal cavity is alleviated. For instance, an average particle diameter of approximately 250 µm or smaller is preferred, an average particle diameter of approximately 5 µm to approximately 250 µm is further preferred, and an average particle diameter of approximately 50 µm to approximately 250 µm is most preferred. It is difficult to provide sustained releasability when the average particle diameter is smaller than 5 µm, while there is an awkward feeling, such as a gritty sensation, in the buccal cavity when the average particle diameter is larger than 250 µm.

Moreover, when dissolution tests are performed by the dissolution testing methods cited in the Japanese Pharmacopoeia using tablets that disintegrate in the buccal cavity comprising the enteric sustained-release fine particles of the present invention, the dissolution rate after 2 hours at a pH of 1.2 is 0 to approximately 25%, preferably 0% to approximately 15%, in dissolution tests. This is because retention time in the stomach is said to be 2 hours and therefore, adverse effects can be reduced by holding dissolution to approximately 25% or less at the estimated pH inside the stomach.

The mixture ratio of water-insoluble polymer, wax, water-soluble polymer, and the like used in the present invention is adjusted as needed so that the time when 50% is dissolved in dissolution tests at a pH of 6.8 is 30 minutes to 5 hours, preferably 30 minutes to 4 hours. This is because as with the above-mentioned controlled dissolution at a pH of 1.2, it is possible to reduce the adverse effects of tamsulosin by adjusting 50% dissolution to these times.

Moreover, the mixture ratio of enterosoluble polymer, plasticizer, and the like used in the present invention is adjusted as needed so that the dissolution rate after two hours in dissolution tests at a pH of 1.2 is 25% or less.

The above-mentioned embodiments of
a. a film-controlled type,
b. a matrix-type, and
c. a combination of a film-controlled type and a matrix-type are preferred in order to realize in full the effect of tamsulosin or its salt as a drug, but optimization of the dissolution rate as follows is also possible.
   - The layer or matrix containing enterosoluble base is the layer that touches the dissolution fluid or at the outermost layer.
   - When the particles contain a water-insoluble substance, the layer containing the water-insoluble substance is at least farther inside the particles than the layer of enterosoluble base.

The "at least" means that many layers can be present and as long as the layer containing the water-insoluble substance is farther inside than the layer containing the enterosoluble base, other layers can be in between these layers.

The enteric sustained-release fine particles that were obtained can be made, together with an appropriate filler and binder as necessary, into tablets that disintegrate in the buccal cavity. The tablets that disintegrate in the buccal cavity can be prepared by conventional methods, and are generally classified as the molded type, wet method type, or the ordinary tablet type, and any of these types can be used. Molded tablets that disintegrate in the buccal cavity are made by filling and drying a solution or suspension of filler, and the like in a mold, as disclosed in Japanese Kokoku Patent No. 62-50445 and Patent No. 2807346. Molded tablets that disintegrate in the buccal cavity comprising enteric sustained-release fine particles can be made by, for instance, filling a solution or suspension of the enteric sustained-release fine particles of the present invention, a filler such as a saccharide, and a binder such as gelatin or agar in a PTP pocket and then removing the moisture by a method such as freeze drying, drying under reduced pressure, or low-temperature drying. Wet method-type tablets that disintegrate in the buccal cavity are made by moistening a filler such as a saccharide, tableting under low pressure, and then drying, as disclosed in Japanese Kokai Patent No. 09-309821 and Japanese Kokai Patent No. 09-309822. Wet-type tablets that disintegrate in the buccal cavity comprising enteric sustained-release fine particles are prepared by, for instance, moistening enteric sustained-release fine particles and a filler such as a saccharide with a small amount of water or a mixture of water and alcohol, molding this moistened mixture under low pressure, and then drying. Ordinary tablets are prepared by going through conventional tableting processes as disclosed in International Publication Pamphlet WO95/20380 and US Patent Application No. 10/142,081. In order to prepare ordinary tablets that disintegrate in the buccal cavity comprising enteric sustained-release fine particles, for instance, a saccharide, the enteric sustained-release fine particles of the present invention, a filler such as a saccharide of low moldability, and/or a melting saccharide are granulated with an aqueous solution of a saccharide of high moldability or a water-soluble polymer, tableted, and then submitted to humidification and drying treatment or heat treatment. It is possible to make tablets that disintegrate in the buccal cavity with improved tablet strength by this treatment. Moreover, as disclosed in Japanese Kokai Patent No. 10-182436 and Japanese Kokoku Patent No. 5-500956, tablets that disintegrate in the buccal cavity can be made by mixing enteric sustained-release fine particles, filler such as saccharide, and disintegrating agent or a foamable component and then tableting this mixture.

The mixture ratio of enteric sustained-release fine particles in the tablet is preferably 1 to 50 w/w%, further preferably 5 to 20 w/w%, per tablet weight. There is a concern that content uniformity cannot be guaranteed if the amount of enteric sustained-release fine particles is less than 1%. Moreover there is a concern that the properties of tablets that disintegrate in the buccal cavity cannot be obtained if the amount of enteric sustained-release fine particles is more than 50%.

There are no special restrictions to the "filler" that is used in the present invention as long as it is a saccharide or sugar alcohol that is pharmaceutically acceptable. For instance, the saccharides of poor moldability in International Publication Pamphlet WO95/20380, specifically xylitol, erythritol, glucose, mannitol, sucrose, and lactose, are preferred, and mamitol, lactose, and erythritol are further preferred. Moreover, one or a combination of two or more of these saccharides can be used.

The saccharide of good moldability or water-soluble polymer substance disclosed in International Publication Pamphlet WO95/20380 is selected as the "binder" used in the present invention. Examples of saccharides of good moldability are maltose (preferably *ame* [maltose syrup] powder (maltose content of 83% or higher)), trehalose, sorbitol, and maltitol, and maltose and trehalose are preferred. Moreover, examples of water-soluble polymer substances are hydroxypropyl cellulose, hydroxypropylmethyl cellulose, polyvinyl pyrrolidone, polyvinyl alcohol, gum arabic powder, gelatin, and pullulan. One or a combination of two or more of these "binders" can be used.

The amount of "filler" used in the present invention is adjusted as needed in accordance with the amount of enteric sustained-release fine particles added and/or the size of the tablet. Normally this amount is preferably 20 to 1,000 mg, further preferably 50 to 500 mg, still further preferably 100 to 400 mg, per 1 tablet. Moreover, in terms of tablet weight it is preferably 30 to 99.5 w/w%, further preferably 50 to 95 w/w%. If the amount of filler added is less than 20 mg, or less than 30 w/w% in terms of tablet weight, there is a possibility that good properties will not be obtained, with a variety of problems developing that include delayed disintegration, when this is made into tablets that disintegrate in the buccal cavity. There is a concern that it will not be possible to realize sufficient disintegration in the buccal cavity. Moreover, if it is more than 1,000 mg, there may also be too much filler in terms of the amount of saliva in the buccal cavity and the tablet will leave uncomfortable feeling in the mouth.

Moreover, normally the amount of "binder" used in the present invention is preferably 0.5 to 25 w/w%, further preferably 2 to 20 w/w%, still further preferably 5 to 10 w/w%, per the weight of the enteric sustained-release fine particles and/or "filler" that is used in the present invention, or 1 to 20 w/w% of the entire pharmaceutical preparation. There is a concern that if it is less than 0.5 w/w% per the weight of the "filler", or if it is less than 1 w/w% of the entire pharmaceutical preparation, its function as a binder will not be realized in full. Moreover, there is a possibility that if there is more than 25 w/w% per the weight of the "filler" or more than 20 w/w% of the entire pharmaceutical preparation, good properties will not be obtained, with a variety of problems developing that include delayed disintegration, when it is made into tablets that disintegrate in the buccal cavity. The mixture ratio of "binder" to "filler" used in the present invention is preferably 99.5:0.5 to 75:25, further preferably 98:2 to 80:20.

A variety of additives that are pharmaceutically acceptable and used as additives can be added in addition to the "filler" used in the present invention. These additives can also be added together with the filler when the enteric sustained-release fine particles are granulated, or they can be mixed with the composition of the present invention when it is tableted. Diluent (extender), disintegrating agent, binder, sour flavoring, foaming agent, artificial sweetener, fragrance, lubricant, coloring agent, and stabilizer are given as examples of these additives. One or a combination of two or more of these additives can be used. Moreover, there are no special restrictions to the amount that is added as long as it is within a range normally used by experts pharmaceutically and it is an amount with which the results of the present invention are not compromised.

Crystalline cellulose and the like can be given as the diluent.

Starches such as corn starch and the like, carmellose calcium, partly pregelatinized starch, crospovidone, and low-substituted hydroxypropyl cellulose are examples of disintegrating agents. Citric acid, tartaric acid, and malic acid are examples of sour flavorings. Sodium bicarbonate is an example of a foaming agent. Saccharine sodium, dipotassium glycyrrhizinate, aspartame, stevia, and somatin are examples of artificial sweeteners. Lemon, lemon-line, orange, and menthol are examples of flavorings. Calcium stearate, magnesium stearate, sucrose fatty acid ester, polyethylene glycol, talc, and stearic acid are examples of lubricants. Food colorings, such as yellow food coloring No. 5, red food coloring No. 2, blue food coloring No. 2 and the like; food lake coloring; and iron oxide red are examples of coloring agents. The stabilizer is selected for each drug after a variety of tests have been performed. One or a combination of two or more of these additives can be added as needed in appropriate amounts.

With respect to dissolution when the enteric sustained-release fine particles of the present invention are contained in tablets that disintegrate in the buccal cavity, when dissolution tests are performed in accordance with the dissolution test method in the Japanese Pharmacopoeia, the dissolution rate after 2 hours in dissolution tests at a pH of 1.2 is 25% or less, preferably 15% or less, and the time when there is 50% dissolution in dissolution tests at a pH of 6.8 is 30 minutes to 5 hours, preferably 30 minutes to 4 hours. This dissolution is dissolution suitable for tamsulosin or its salts in terms of the adverse effects of and in terms of the efficacy of tamsulosin or its salt. Dissolution of the enteric sustained-release fine particles and dissolution of tablets that disintegrate in the buccal cavity containing enteric sustained-release fine particles are approximately the same, and dissolution of either can be measured in order to confirm the results of the enteric sustained-release fine particles of the present invention.

The process of manufacturing enteric sustained-release fine particles of the present invention, and the process of producing tablets that disintegrate in the buccal cavity containing enteric sustained-release fine particles of the present invention, particularly the manufacturing conditions and the like, will now be described in detail:

The method of manufacturing tablets that disintegrate in the buccal cavity containing enteric sustained-release fine particles of the present invention is described in terms of (a) the process of manufacturing enteric sustained-release fine particles containing tamsulosin or its salt with which the dissolution speed of the tamsulosin or its salt is controlled, and (b) the process of making the "enteric sustained-release fine particles" and "filler," and when necessary, "binder," into tablets that disintegrate in the buccal cavity.

### Process (a): Process of manufacturing enteric sustained-release fine particles

The enteric sustained-release fine particles are manufactured using a conventional device. There are no special restrictions to this method and it can be selected as needed as long as dissolution is controlled as intended.

Enteric sustained-release fine particles of the film-controlled type are, for instance, made by coating a core of commercial crystalline cellulose grains or a conventiopal crystalline filler having an average particle diameter of approximately 5 µm to approximately 150 µm, specifically crystalline lactose, granular sugar, sodium chloride, corn starch, and the like, with a drug using a binder, such as hydroxypropyl cellulose, and then further coating this product with a polymer substance, such as water-insoluble polymer substance or a wax-like substance, to make sustained-release fine particles. It is also possible to make sustained-release fine particles by preparing a core made from a drug, crystalline cellulose, and the like by the high shear granulation method, tumbling granulation method, or spray drying method and then coating this with a water-insoluble polymer or wax. There are no restrictions to the amount of water-insoluble polymer or wax as long as the target dissolution speed can be realized and it is, for instance, 5 to 50%, preferably 10 to 25%, per core containing drug. Enteric sustained-release fine particles of the film-controlled type can be obtained by coating the sustained-release fine particles that have been obtained with an enterosoluble substance. There are no restrictions to the amount of enterosoluble substance as long as the target dissolution speed can be realized and it is, for instance, 5 to 100%, preferably 20 to 80%, further preferably 25 to 50%, per core containing drug.

Enteric sustained-release fine particles of the matrix type are, for instance, obtained by coating a core of commercial crystalline cellulose grains or a conventional crystalline filler, specifically crystalline lactose, granular sugar, sodium chloride, corn starch, and the like, with a drug and an enterosoluble substance to make enteric sustained-release fine particles. It is also possible to make enteric sustained-release fine particles by the high shear granulation method or tumbling agitation method after adding the enterosoluble substance to the drug and microcrystalline cellulose. Enteric sustained-release fine particles can also be made by spray drying or spray cooling a solution, suspension, or melt of the drug and enterosoluble substance using an appropriate device, such as spray dryer. There are no restrictions to the amount of substance that forms the matrix at this time as long as the target dissolution speed can be accomplished and it is, for instance, 10 to 200 parts, preferably 50 to 100 parts, per 1 part of drug.

Enteric sustained-release fine particles of the combination type are obtained by coating both a core of commercial crystalline cellulose grains or a conventional crystalline filler, specifically crystalline lactose, granular sugar, sodium chloride, corn starch, and the like, with a drug and a polymer substance, such as a water-insoluble polymer substance or wax-like substance, to make sustained-release fine particles. It is also possible to make sustained-release fine particles by the high shear granulation method or tumbling fluidized granulation method after adding water-insoluble substance to the drug and microcrystalline cellulose. Sustained release fine particles can also be made by spray drying a solution, suspension, or melt of a drug and a water-insoluble substance using an appropriate device, such as a spray dryer. An enterosoluble substance is further coated on these sustained-release fine particles to make enteric sustained-release fine particles of the combination type.

For instance, a fluidized bed granulator is selected for coating. Temperature is set, and the spraying liquid volume, drying air volume, and the like are set so that when coating is performed using water, temperature is approximately 40°C to approximately 60°C, and when coating is performed using an organic solvent, temperature is approximately 30°C to approximately 60°C. The concentration of drug that is to be coated, percentage and amount of polymer substance, and the like can be adjusted as needed in accordance with the targeted speed of dissolution.

The enteric sustained-release fine particles are produced by coating sustained-release fine particles with an enterosoluble polymer by conventional methods. There are no special restrictions to this method and it can be selected as needed as long as dissolution is controlled as intended. For instance, a fluidized bed granulator is selected. Temperature is set, and the spraying liquid volume, drying air volume, and the like are set so that when coating is performed using water, temperature is approximately 40°C to approximately 60°C, and when coating is performed using an organic solvent, temperature is approximately 30°C to approximately 60°C. The concentration of drug that is to be coated, percentage and amount of polymer substance, and the like, can be adjusted as needed in accordance with the targeted speed of dissolution.

### Process (b): Process of making tablets that disintegrate in the buccal cavity

The "enteric sustained-release fine particles" and "filler," "binder" when necessary, are made into tablets that disintegrate in the buccal cavity by conventional methods.

The molded type of tablet that disintegrate in the buccal cavity can be made by filling a solution or suspension of enteric sustained-release fine particles, filler such as saccharide, and binder such as gelatin or agar in a PTP pocket and then removing the water by a method such as freeze drying, drying under reduced pressure, or low-temperature ventilation drying, as disclosed in Japanese Kokoku Patent No. 62-50445 and Japanese Patent No. 2807346. The type of tablet that disintegrates in the buccal cavity made by the wet method can be obtained by moistening the enteric sustained-release fine particles, filler such as saccharide with a small amount of water or a mixture of water and alcohol, tableting this moist mixture under low pressure, and then drying, as disclosed in Japanese Kokai Patent No. 09-309821 and Japanese Kokai Patent No. 09-309822. The conventional tableted type of tablet that disintegrates in the buccal cavity can be made by granulating enteric sustained-release fine particles and filler such as a saccharide of poor moldability, and/or molten saccharide with an aqueous solution of a saccharide with good moldability or a water-soluble polymer, tableting, and then improving tablet strength by humidification and drying treatment or heat treatment, as disclosed in International Publication Pamphlet WO95/20380 and International Publication Pamphlet WO02/092057. Moreover, the conventional tableted type that disintegrates in the buccal cavity can also be made by mixing enteric sustained-release fine particles, filler such as saccharide, and disintegrating agent or foamable component and then tableting, as disclosed in Japanese Kokai Patent No. 10-182436 and Japanese National Publication No. 5-500956.

Consequently, it is possible to make various types of tablets that disintegrate in the buccal cavity as long as the enteric sustained-release fine particles of the present invention are used.

The "drying" method can be selected from any method commonly used in this field, including freeze drying, drying under reduced pressure, and drying by low-temperature ventilation, as long as it is possible to remove the water.

Fluidized bed granulation, high shear granulation, and tumbling granulation are examples of methods that can be selected for "granulation". Of these, fluidized bed granulation is preferred in terms of productivity. In this case, it is preferred that the saccharide is pulverized with an appropriate pulverizing device, such as a hammer mill, sample mill, or pin mill, if the average particle diameter of the saccharide that is to be added is larger than the average particle diameter of the enteric sustained-release fine particles.

"Tableting" is performed by conventional methods. This "tableting" can be performed using, for instance, an ordinary tableting machine, such as a single tableting machine or a rotary tableting machine, after adding the necessary additives, including a lubricant such as calcium stearate, to the above-mentioned "granulated product." Moreover, the above-mentioned " granulated product" can also be made into tablets using an external-lubricating tableting machine. Usually, tableting pressure of approximately 25 to approximately 800 kg/punch is preferred, approximately 50 to approximately 500 kg/punch is further preferred, approximately 50 to approximately 300 kg/punch is most preferred.

### Brief Description of the Drawings

Figure 1 is the results of dissolution tests on the tablets and sustained-release fine particles in Example 1 using the 1st fluid for the disintegration test of the Japanese Pharmacopoeia.
Figure 2 is the results of dissolution tests on the tablets and sustained-release fine particles in Example 1 using the 2nd fluid for disintegration tests of the Japanese Pharmacopoeia

### Description of the Preferred Embodiments

The present invention will now be explained in further detail while referring to examples, but the present invention is not limited to these examples.

### Methods of Evaluating Tablets that Disintegrate in the Buccal Cavity

[Hardness tests] Determinations were performed using a Schleuniger tablet hardness meter (Schleuniger Co., Ltd.). The tests are performed with 5 tablets and the mean value is shown. Tablet hardness is represented by the force needed to break the tablet (units kp). A larger number indicates a stronger tablet.

[Friability] Determinations were performed using a friability tester (model PTFR-A, Pharma Test Co.). Friability is found using 6 or 6.5 g tablets. It is represented by the percentage weight loss of a tablet after being turned 100 rounds at a turning speed of 25 rpm. A smaller value indicates a stronger tablet surface.

[Disintegration in the buccal cavity tests] Healthy adult males place the tablet of the present invention inside their buccal cavity without having any water inside their mouth and the time until the tablet is completely disintegrated and dissolved by saliva only is determined.

[Dissolution tests] These were performed in accordance with Dissolution Testing Method 2 in accordance with the 12th Revised Edition of the Japanese Pharmacopoeia. Example 1

Eighty grams of tamsulosin hydrochloride and 80 g of hydroxypropylmethyl cellulose (TC5E, Shin-Etsu Chemical Co., Ltd.) were dissolved in a mixture of 304 g of purified water and 2,736 g of methanol. Four-thousand grams of Celphere 102Y (brand name, Asahi Kasei Corp.) were introduced to a fluidized bed granulator (Freund Industries, FLO-5) and coated with this solution by the side spraying method (spraying liquid volume of 100 g/min, spraying air pressure of 4 kg/cm², product temperature of 40°C, inlet temperature of 80°C) to obtain tamsulosin hydrochloride particles. Separately, 533 g of ethyl cellulose (Nisshin Chemical Co.) and 187 g of [hydroxypropylmethyl cellulose] TC5E (brand name, Shin-Etsu Chemical Co., Ltd.) were dissolved in a mixture of 698 g of purified water and 22,582 g of methanol. Four-thousand grams of tamsulosin hydrochloride particles were introduced to a fluidized bed granulator (Freund Industries, FLO-5) and coated with this solution by the side spraying method (spraying liquid volume of 40 g/min, spraying air pressure of 4 kg/cm², product temperature of 50°C, inlet temperature of 60°C) to obtain sustained-release fine particles. Four-thousand grams of these sustained-release fine particles were introduced to a fluidized bed granulator (Freund Industries, FLO-5) and coated with a mixture of 2,000 g of Aquacoat (brand name, Asahi Kasei Corp.), 4,000 g of Eudragit L30D55 (brand name, Röhm GmbH), 667 g of Eudragit NE30D (brand name, Röhm GmbH), and 6,667 g of purified water by the side spraying method (spraying liquid volume of 40 g/min, spraying air pressure of 4 kg/cm², product temperature of 40°C, inlet temperature of 60°C) to obtain enteric sustained-release fine particles. The average particle diameter of the enteric sustained-release fine particles of the present invention was 180 µm.

Then 368 g of these enteric sustained-release fine particles, 2,560 g of mannitol (Towa Kasei Co., Ltd.), and 640 g of lactose (Domomilk) were granulated (spraying liquid volume of 200 g/min, spraying air pressure of 1.5 kg/cm², product temperature of 29°C, inlet temperature of 80°C, spraying cycle of 10 seconds spraying-30 seconds drying) with an aqueous 40 w/w% solution containing 400 g of maltose (Hayashibara Co., Ltd., brand name Sunmalt S) in a fluidized bed granulator (Freund Industries, FLO-5) to obtain a granulated product.

After further mixing 32 g of calcium stearate with the granulated product that was obtained, 200 mg tablets containing 0.2 mg of tamsulosin hydrochloride per tablet were made under a tableting pressure of 100 kg/punch and an initial hardness of 1.0 kp using a rotary tableting machine. Next, these tablets were kept for 18 hours while heating and humidifying at 25°C/75% RH using a thermostatic chamber at constant humidity (Tabaiespec Co., Ltd., PR-35C). Then they were dried for 3 hours at 30°C and 40% RH. The tablets that were obtained showed a hardness of 5.9 kp, friability of 0.8% and disintegration time in the buccal cavity of 20 seconds.

### Experiment 1 (Dissolution test)

Dissolution tests were conducted on the enteric sustained-release fine particles and tablets obtained in Example 1. The experimental conditions were 100 rpm by the paddle method, and 500 ml each of the 1st fluid (pH 1.2) and 2nd fluid (pH 6.8) of the disintegration test method of the Japanese Pharmacopoeia were used as the test fluids.

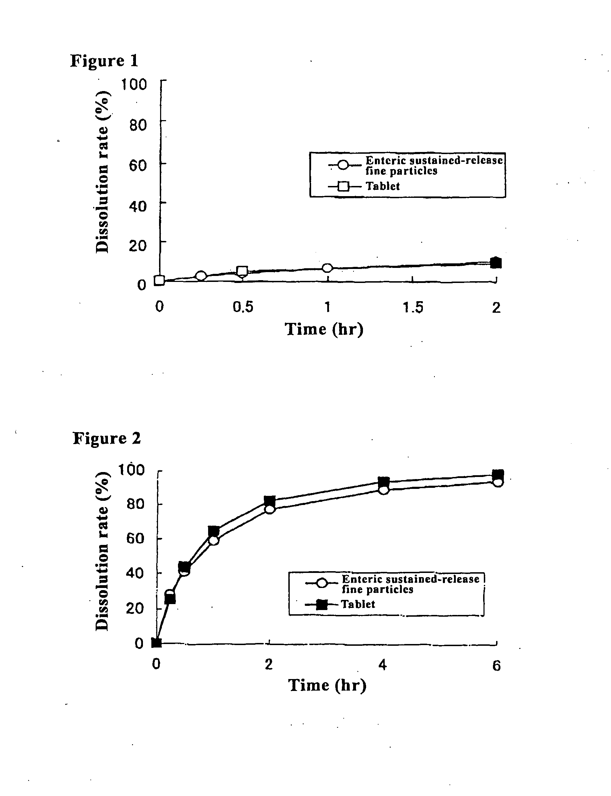

As a result of the tests, the dissolution rate of the enteric sustained-release fine particles and tablets was 10% and 9%, respectively, 2 hours after starting the dissolution test in test fluid at a pH of 1.2. Moreover, the dissolution rate of the enteric sustained-release fine particles and tablets in test fluid at a pH of 6.8 was, respectively, 27% and 25% fifteen minutes after starting the test, 58% and 64% one hour after starting the test, and 89% and 93% four hours after starting the test (Figures 1 and 2).

### Example 2

Eighty grams of tamsulosin hydrochloride and 80 g of hydroxypropylmethyl cellulose (TC5E, Shin-Etsu Chemical Co., Ltd.) were dissolved in a mixture of 304 g of purified water and 2,736 g of methanol. Four-thousand grams of Celphere 102 (brand name, Asahi Kasei Corp., average particle diameter of approximately 127 µm, particle diameter of approximately 50 to approximately 150 µm) were introduced to a fluidized bed granulator (Freund Industries, FLO-5) and coated with this solution by the side spraying method (spraying liquid volume of 100 g/min, spraying air pressure of 4 kg/cm², product temperature of 40°C, inlet temperature of 80°C) to obtain tamsulosin hydrochloride particles.

Separately, 43.7 g of ethyl cellulose (Nisshin Chemical Co.) and 12.3 g of hydroxypropylmethyl cellulose (TC5E, brand name, Shin-Etsu Chemical Co., Ltd.) were dissolved in a mixture of 43.9 g of purified water and 833.4 g of methanol. Four-hundred grams of tamsulosin hydrochloride particles were introduced.to a fluidized bed granulator (Freund Industries, uni-glatt) and coated with this solution by the side spraying method (spraying liquid volume of 6 g/min, spraying air pressure of 4 kg/cm², product temperature of 40°C, inlet temperature of 63°C) to obtain sustained-release fine particles.

Three-hundred grams of these sustained-release fine particles were further introduced to a fluidized bed granulator (Freund Industries, uni-glatt) and coated with a mixture of 90 g of Aquacoat (brand name, Asahi Kasei Corp.), 180 g of Eudragit L30D55 (brand name, Röhm GmbH), 30 g of Eudragit NE30D (brand name, Röhm GmbH), and 300 g of purified water by the side spraying method (spraying liquid volume of 6 g/min, spraying air pressure of 3 kg/cm², product temperature of 40°C, inlet temperature of 75.5°C) to obtain enteric sustained-release fine particles.

The average particle diameter of the enteric sustained-release fine particles of the present invention was 157 µm. When drug dissolution at a pH of 1.2 (Japanese Pharmacopoeia 1st fluid), it was 4.2% two hours after starting the test. When drug dissolution at a pH of 6.8 (Japanese Pharmacopoeia 2nd fluid) was evaluated, it was 30.0% one hour after starting the test, 50.0% two hours after starting the test, and 79.4% six hours after starting the test. Then 92.5 g of these enteric sustained-release fine particles, 568.2 g of mannitol (Towa Kasei Co., Ltd.) that had been pulverized with a pin mill pulverizing device (Hosokawa Micron), 142.1 g of lactose (Domomilk), and 72 g of erythritol (Nikken Chemicals Co., Ltd.) were granulated (spraying liquid volume of 15 g/min, spraying air pressure of 0.5 kg/cm², product temperature of 40°C, inlet temperature of 70°C, spraying cycle of 15 seconds spraying-30 seconds drying) with an aqueous 5 w/w% solution containing 18 g of copolyvidone (BASF, brand name Kollidon VA64) in a fluidized bed granulator (Freund Industries, uni-glatt) to obtain a granulated product.

After further mixing 7.2 g of calcium stearate with the granulated product that was obtained, 300 mg of tablets containing 0.4 mg of tamsulosin hydrochloride per tablet were made under an initial hardness of 0.6 kp using a rotary tableting machine. Next, these tablets were heated for 13 minutes at 120°C using a program oven (Model MOV-112P, Sanyo) and then set aside to cool for 30 minutes at room temperature to obtain tablets that disintegrate in the buccal cavity. The tablets that were obtained showed a hardness of 6.8 kp (n = 5), friability of 0.28% (100 rounds) and disintegration time in the buccal cavity of 27 seconds (n = 1).

When drug dissolution at a pH of 1.2 (Japanese Pharmacopoeia 1 st fluid) was evaluated, it was 4.9% two hours after starting the test. When drug dissolution was evaluated at a pH of 6.8 (Japanese Pharmacopoeia 2nd fluid) was evaluated, it was 31.1 % one hour after starting the test, 52.8% two hours after starting the test, and 88.8% six hours after starting the test.

### Example 3

A mixture of 5.25 g of tamsulosin hydrochloride, 1,102.5 g of Aquacoat (brand name, Asahi Kasei Corp.), 122.5 g of Eudragit NE30D (brand name, Röhm GmbH), and 525 g of purified water was prepared. Then 350 g of Celphere 102 (brand name, Asahi Kasei Corp., average particle diameter of approximately 127 µm, particle diameter of approximately 50 to approximately 150 µm) were introduced to a fluidized bed granulator (Freund Industries, uni-glatt) and coated with this solution by the side spraying method (spraying liquid volume of 7 g/min, spraying air pressure of 3.0 kg/cm², product temperature of 45°C, inlet temperature of 80°C) to obtain sustained-release fine particles that contain tamsulosin hydrochloride.

Then 350 g of these sustained-release fine particles were further introduced to a fluidized bed granulator (Freund Industries, uni-glatt) and coated with a mixture of 117 g of Aquacoat (brand name, Asahi Kasei Corp.), 408 g of Eudragit L30D55 (brand name, Röhm GmbH), 58 g of Eudragit NE30D (brand name, Röhm GmbH), and 583 g of purified water by the side spraying method (spraying liquid volume of 6 g/min, spraying air pressure of 3.5 kg/cm², product temperature of 43°C, inlet temperature of 75°C) to obtain enteric sustained-release fine particles.

The average particle diameter of the enteric sustained-release fine particles of the present invention was 202 µm. Moreover, when drug dissolution was evaluated at a pH of 1.2 (Japanese Pharmacopoeia 1st fluid), it was 13% two hours after starting the test. When drug dissolution at a pH of 6.8 (Japanese Pharmacopoeia 2nd fluid) was evaluated, it was 39% one hour after starting the test, 55% three hours after starting the test, and 64% six hours after starting the test.

Then 38 g of these enteric sustained-release fine particles, 159.6 g of mannitol (Towa Kasei Co., Ltd.), and 159.6 g of lactose (Domomilk), were granulated (spraying liquid volume of 15 g/min, spraying air pressure of 1.5 kg/cm², product temperature of 35°C, inlet temperature of 90°C, spraying cycle of 60 seconds spraying-60 seconds drying) with an aqueous 30 w/w% solution containing 19 g of maltose (Hayashibara Co., Ltd., brand name; Sunmalt S) in a fluidized bed granulator (Freund Industries, uni-glatt) to obtain a granulated product.

After further mixing 3.8 g of calcium stearate with the granulated product that was obtained, 380 mg of tablets containing 0.2 mg of tamsulosin hydrochloride per tablet were made under an initial hardness of 1.3 kp using a rotary tableting machine. Next, these tablets were kept for 18 hours while heating and humidifying at 25°C/70% RH using a thermostatic chamber at constant humidity (Tabaiespec Co., Ltd., built-in chamber TBL-2N1AGP). The tablets were then dried for 4 hours at 30°C and 40% RH to obtain tablets of the present invention. The tablets that were obtained showed a hardness of 4.3 kp, friability of 0.7% and disintegration time in the buccal cavity of 25 seconds.

When drug dissolution at a pH of 1.2 (Japanese Pharmacopoeia 1 st fluid) was evaluated, it was 15% two hours after starting the test. When drug dissolution at a pH of 6.8 (Japanese Pharmacopoeia 2nd fluid) was evaluated, it was 37% one hour after starting the test, 53% three hours after starting the test, and 63% six hours after starting the test.

### Example 4

After dispersing 1 part tamsulosin hydrochloride in 99 parts molten stearic acid, spray congealing (liquid speed of 47 g/minute, atomizer disk turning speed of 2,500 rpm) was performed using a spray dryer (Ohkawara Kakohki Co., Ltd., DL-8) to obtain enteric sustained-release fine particles. The average particle diameter of the enteric sustained-release fine particles of the present invention was 198 µm. Moreover, when drug dissolution was evaluated at a pH of 1.2 (Japanese Pharmacopoeia 1st fluid), it was 10% two hours after starting the test. Furthermore, when drug dissolution at a pH of 6.8 (Japanese Pharmacopoeia 2nd fluid) was evaluated, it was 14.9% one hour after starting the test, 46% four hours after starting the test, and 61% six hours after starting the test.

A solid pharmaceutical preparation was obtained by drying (25°C, -760 mmHg) 147 mg of a suspension made from 10 parts of these enteric sustained-release fine particles, 0.5 part agar, 90 parts lactose, 95.5 parts mannitol, 4 parts citric acid, and 60 parts purified water in the mold. The tablets that were obtained had a hardness of 2.5 kp and a disintegration time in the buccal cavity of 6 seconds.

Moreover, when drug dissolution at a pH of 1.2 (Japanese Pharmacopoeia 1st fluid) of these tablets was evaluated, it was 13. 1 % two hours after starting the test. Furthermore, when drug dissolution at a pH of 6.8 (Japanese Pharmacopoeia 2nd fluid) was evaluated, it was 18% one hour after starting the test, 47% four hours after starting the test, and 62% six hours after starting the test.

### Industrial Applicability

The present invention presents tablets that disintegrate in the buccal cavity of tamsulosin hydrochloride or its salt with which it is possible to inhibit the adverse effect of orthostatic hypotension attributed to a sudden rise in plasma concentrations by making the tamsulosin hydrochloride or its salt into enteric sustained-release fine particles. Moreover, it presents tablets that disintegrate in the buccal cavity of tamsulosin hydrochloride or its salt that have been given what at a glance are opposing qualities in that although their property of being tablets that are disintegrated in the buccal cavity is not compromised and they are quickly disintegrated and dissolved in the buccal cavity, they have sustained releasability as a result of being made into enteric sustained-release fine particles the size of which does not produce a gritty sensation in the buccal cavity.

## Claims

1. Enteric sustained-release fine particles for tablets that disintegrate in the buccal cavity, which comprise (1) tamsulosin or its salt and at least (2) an enterosoluble substance, and when necessary contain (3) a water-insoluble substance, and which have the following characteristics:
1) A particle diameter of approximately 5 to 250 µm
2) When dissolution tests are performed on tablets that disintegrate in the buccal cavity containing these particles by dissolution testing methods cited in the Japanese Pharmacopoeia,
a) the dissolution rate of tamsulosin or its salt at a pH of 1.2 two hours after starting tests is 25% or less
b) the time when 50% of the tamsulosin or its salt has dissolved at a pH of 6.8 is 0.5 to 5 hours.

2. The enteric sustained-release fine particles for tablets that disintegrate in the buccal cavity according to claim 1, wherein the enterosoluble substance is an enterosoluble polymer and/or higher fatty acid.

3. The enteric sustained-release fine particles for tablets that disintegrate in the buccal cavity according to claim 2, wherein the water-insoluble substance is a water-insoluble polymer and/or wax.

4. The enteric sustained-release fine particles for tablets that disintegrate in the buccal cavity according to claim 3, **characterized in that** dissolution of the tamsulosin or its salt is controlled by a controlling film and/or matrix.

5. The enteric sustained-release fine particles according to claim 4, wherein a layer or matrix containing an enterosoluble base is the layer that touches the dissolution fluid or the outermost layer, and the layer containing the water-insoluble substance is farther inside the particles than at least the layer of the enterosoluble base.

6. A method of producing enteric sustained-release fine particles for tablets that disintegrate in the buccal cavity, which comprise (1) tamsulosin or its salt and at least (2) an enterosoluble substance, and when necessary contain (3) a water-insoluble substance, and which have the following characteristics:
1) A particle diameter of approximately 5 to 250 µm
2) When dissolution tests are perfomied on tablets that disintegrate in the buccal cavity containing these particles by dissolution testing methods cited in the Japanese Pharmacopoeia,
a) the dissolution rate of tamsulosin or its salt at a pH of 1.2 two hours after starting tests is 25% or less
b) the time when 50% of the tamsulosin or its salt has dissolved at a pH of 6.8 is 0.5 to 5 hours.
